(19) Europäisches Patentamt
European Patent Office
Office européen des brevets

(11) Publication number: 0 258 006 A2

# (12) EUROPEAN PATENT APPLICATION

(21) Application number: 87307404.1

(22) Date of filing: 21.08.87

(51) Int. Cl.[4]: C 12 P 21/00
C 12 N 15/00, G 01 N 33/577

(30) Priority: 22.08.86 US 899163

(43) Date of publication of application:
02.03.88 Bulletin 88/09

(84) Designated Contracting States:
BE CH DE ES FR GB IT LI LU NL SE

(71) Applicant: WESTINGHOUSE ELECTRIC CORPORATION
Westinghouse Building Gateway Center
Pittsburgh Pennsylvania 15222 (US)

(72) Inventor: Hunter, Kenneth W.
8600 Fox Run
Potomac Maryland 20854 (US)

(74) Representative: Marchant, James Ian et al
Elkington and Fife High Holborn House 52/54 High Holborn
London WC1V 6SH (GB)

The applicant has filed a statement in accordance with Rule 28 (4) EPC (issue of a sample only to an expert). Accession number(s) of the deposit(s): ATCC HB 9183, ATCC HB 9184.

## (54) Monoclonal antibodies reactive with chlorinated dibenzo-p-dioxins and method of preparing and using same.

(57) Monoclonal antibodies that react with chlorinated dibenzo-p-dioxins, particularly, 2,3,7,8-tetrachlorodibenzo-p-dioxin. Also a method of producing such antibodies by producing an immunogenic conjugate of a chlorinated dibenzo-p-dioxin and a macromolecule carrier, immunizing an animal with the conjugate, obtaining antibody-producing cells from the animal, fusing the cells with tumor cells to produce hybridomas, selecting from among the hybridomas at least one that produces antibodies reactive with the chlorinated dioxin, and recovering the antibodies. Also analytic, diagnostic, investigational, separatory and other methods of using the antibodies, and compositions containing the antibodies for such uses.

EP 0 258 006 A2

**Description**

Monoclonal Antibodies Reactive with Chlorinated Dibenzo-p-Dioxins and Method of Preparing and Using Same

This invention relates to monoclonal antibodies that react with chlorinated dibenzo-p-dioxins and particularly to monoclonal antibodies that react with the toxic chemical 2,3,7,8-tetrachlorodibenzo-p-dioxin (2,3,7,8-TCDD). The invention is directed to the antibodies, processes of preparing the antibodies, analytic, diagnostic, investigational, separatory, and other methods of using the antibodies, and compositions containing the antibodies for such uses.

The chlorinated dibenzo-p-dioxins (chlorinated dioxins) are a class of widespread environmental pollutants. They occur as contaminants in commercial chlorophenols and chlorophenoxyacids. One of the chlorinated dibenzo-p-dioxins, 2,3,7,8-TCDD, is one of the most toxic man-made chemicals known. Other highly toxic chlorinated dibenzo-p-dioxins include the 1,2,3,7,8-pentachloro-, 1,2,3,6,7,8-hexachloro-, and 1,2,3,7,8,9-hexachloro isomers. Only a few of the many possible chlorinated dioxins have thus far undergone toxicity testing.

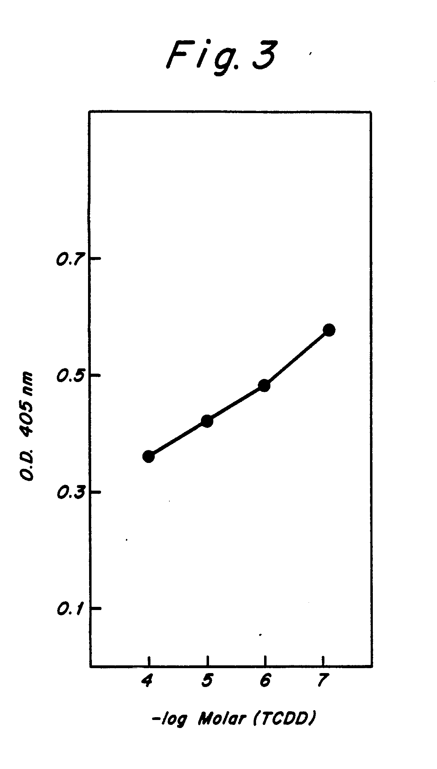

2,3,7,8-TCDD is an unwanted by-product of many chemical processes. Although this chemical compound is not of practical use, its extreme toxicity makes it one of the most important chemicals known. The structure of 2,3,7,8-TCDD is shown in Fig. 1. 2,3,7,8-TCDD is but one of many dioxin isomers, but because of its extraordinary toxicity for man and animals, the term "dioxin" is often used to describe the action of the single isomer, 2,3,7,8-TCDD.

Although there is some controversy over the long-term human health effects of exposure to low levels of 2,3,7,8-TCDD, environmental and public health officials are nevertheless very concerned about human exposure to the chemical because of its acute toxicity. 2,3,7,8-TCDD is strictly regulated by the Environmental Protection Agency (EPA) as a priority pollutant. Detection of levels of low as 1 part per trillion (ppt) in soil, air, or water is sufficient to warrant decontamination and cleanup. For example, the discovery of 2,3,7,8-TCDD at sites in Love Canal, New York and Times Beach, Missouri required evacuations of people, quarantine of large areas, and a major, expensive cleanup effort that may take ten years. The importance of 2,3,7,8-TCDD as a toxic chemical has been reviewed by several authors. See Young et al., Environ. Sci. Technol. 17, 530A-540A (1983), Carter et al., Science 188, 738-74C (1975), and Firestone, Ecol. Bull. (Stockholm) 27, 39-52 (1978).

Because 2,3,7,8-TCDD is an environmental and medical problem in extremely low concentrations, it is imperative that very sensitive analytical methods exist for its detection. At the present time, the EPA approved method for 2,3,7,8-TCDD analysis involves an extensive extraction and cleanup procedure for each sample, followed by a combination of gas chromatography and high resolution mass spectrometry (GC-MS) with specific ion monitoring. This method is described in an EPA publication entitled The National Dioxin Study (EPA/600/3-85/019, April 1985).

The GC-MS method has several disadvantages. It is costly, time consuming, and labor intensive. The equipment is complex and expensive, and it requires highly trained operators. It takes about three days to analyze a sample. In addition, although this method has a sensitivity of approximately 1 part per trillion, it is desirable to be able to detect 2,3,7,8-TCDD at significantly lower concentrations.

At least two other methods have been reported for the detection of 2,3,7,8-TCDD. One involved the induction of aryl hydrocarbon hydroxylase (AHH) activity in a rat hepatoma cell line by 2,3,7,8-TCDD. Bradlaw and Casterline, J. Assoc. Off. Anal. Chem., 62, 904-916 (1979). The percent induction of AHH activity by a sample suspected of containing 2,3,7,8-TCDD or other polychlorinated planar organic substances was compared to a standard curve of the percent induction of AHH activity by known concentrations of 2,3,7,8-TCDD. Although this method is faster, cheaper, and easier to use than the GC-MS method, its lack of specificity is a serious disadvantage. It cannot distinguish 2,3,7,8-TCDD from other dioxin isomers or even from many other polychlorinated planar organic compounds.

The second method involves the use of polyclonal antibodies to chlorinated dibenzo-p-dioxins in a radioimmunoassay. See Albro et al., Toxicology and Applied Pharmacology, 50, 137-146 (1979) and Albro et al., Methods in Enzymology, 84, 619-628 (1982). This method is also faster, cheaper, and easier to use than the CG-MS method, and it is much more specific than the AHH induction method. However, it too suffers from significant disadvantages. It does not distinguish among the different isomers of chlorinated dioxins and dibenzo-furans. In addition, although chlorinated dioxins coupled to proteins yield good antibody responses in rabbits, binding specificity can vary significantly from animal to animal. Extensive characterization of sera is required, which is a laborious process.

Several other disadvantages of the Albro et al. method result from the fact that it employs radioactive materials. Elaborate safety precautions must be maintained during the preparation, utilization, and disposal of the radioactive reagents used in the assay. Radioactive reagents also add to the cost of the assay. Finally, the radioactive materials can be unstable and, therefore, can have a limited shelf life.

Still other disadvantages to the assay of Albro et al. result from the use of rabbit antisera as a source of antibodies to the chlorinated dibenzo-p-dioxins. Such antisera contain a complex mixture of antibodies with a multitude of specificities and affinities for the antigen. As a result, an assay employing such antibodies, i.e, polyclonal antibodies, may not be sufficiently specific for the particular isomer of dioxin sought to be detected, such as 2,3,7,8-TCDD. In addition, because of the method used to produce such antibodies--immunization of an animal by the antigen to which antibodies are sought--there can be variability among different batches of the antibodies produced, which can lead to variable results. Also, a living animal is an uncertain source of antibodies.

Thus, the heterogeneity and diversity of polyclonal antibodies, the unpredictability of antibody responses to antigenic stimuli, and the lack of a constant source of the antibodies are factors which seriously limit the practical use of these polyclonal antibodies for clinical or scientific purposes, including their use for the detection and quantification of 2,3,7,8-TCDD.

In contrast to polyclonal antibodies, monoclonal antibodies are derived from a single B lymphocyte clone, which makes them absolutely specific and homogeneous. In 1975, Kohler and Milstein, Nature, 265:495 (1975) first described how monoclonal antibodies directed to sheep red blood cells may be prepared by fusing a specific antibody-producing B lymphocyte with a tumor cell, resulting in an "immortal" self-reproducing hybrid clone (or "hybridoma") that can synthesize, in a test tube (in vitro) or an animal (in vivo), a single, monoclonal antibody. Such a hybridoma is, in effect, a self-reproducing cell "factory" which can produce a potentially limitless supply of an antibody with single, pre-defined specificity.

One attempt to make monoclonal antibodies to 2,3,7,8-TCDD has been reported in the literature. Kennel et al., Toxicology and Applied Pharmacology, 82, 256-263 (1986). A thyroglobulin conjugate of dioxin (thyroglobulin-2 adipamide, 3,7,8-trichlorodibenzo-p-dioxin) (TG-TCDD) was used to immunize mice. Hybridomas were produced by cell fusion between immune spleen cells and mouse myelomas. The authors identified several monoclonal antibodies that bound to a bovine serum albumen (BSA) conjugate of dioxin (BSA-2-adipamide,3,7,8-trichlorodibenzo-p-dioxin, BSA-TCDD) but reported no data showing that they found monoclonal antibodies to 2,3,7,8-TCDD alone. In fact, the entire thrust of the article was to report the development of a rapid, solid-phase assay to identify and characterize monoclonal antibodies that bind to dioxins linked to protein carriers. Such monoclonal antibodies that bind to dioxins linked to carrier proteins, but not to free dioxins, have no utility for the quantitative analysis of dioxins. It is known by those skilled in the art that monoclonal antibodies raised against small chemical compounds conjugated to carrier proteins tend to recognize not only the structure of the chemical, but also the structures of the linker between chemical and protein, and even structures on the protein adjacent to the side of attachment of the linker. The result of such recognition of ancillary structures is the inability, or markedly reduced ability, of the monoclonal antibody to bind to the free chemical. This problem must be overcome if monoclonal antibodies to chemicals are to be useful for quantitative analysis.

The preparation has been undertaken of novel, self-reproducing cell lines which synthesized monoclonal antibodies reactive in any way with free chlorinated dioxins, particularly free 2,3,7,8-TCDD, since such antibodies, if, indeed, they could be produced, would satisfy a number of critical needs not fulfilled by existing polyclonal antibody technology. Like polyclonal antibodies to 2,3,7,8-TCDD, such monoclonal antibodies would bind to 2,3,7,8-TCDD, thus allowing the detection of this very toxic chemical by a variety of rapid, simple, and cost effective immunoassay techniques. However, they would be more useful than polyclonal antibodies as detection reagents because of their exquisite specificity. Likewise, this specificity would also render such monoclonal antibodies more useful for immunological and biochemical studies of 2,3,7,8-TCDD, for affinity purification of 2,3,7,8-TCDD, and for the neutralization and/or removal of 2,3,7,8-TCDD from water or other 2,3,7,8-TCDD containing materials. Furthermore, unlike conventional polyclonal antibodies, monoclonal antibodies reactive with 2,3,7,8-TCDD could be produced in a potentially limitless and homogeneous supply, thus avoiding the problems imposed by the low immunogenicity of 2,3,7,8-TCDD/protein conjugates, the variability of polyclonal antibody response, and the resulting limitations in achievable levels or supplies of anti-2,3,7,8-TCDD antibodies available for environmental and other applications.

Accordingly, the present invention resides in a monoclonal antibody reactive with a chlorinated dibenzo-p-dioxin and a hybridoma that produces such an antibody. Preferably, the monoclonal antibody reacts with 2,3,7,8-TCDD.

The invention further includes a method of producing monoclonal antibodies reactive with a chlorinated dibenzo-p-dioxin by producing an immunogenic conjugate of the chlorinated dibenzo-p-dioxin and a macromolecule carrier, immunizing an animal with the conjugate, obtaining antibody-producing cells from the animal, fusing the cells with tumor cells to produce hybridomas, selecting from among the hybridomas at least one that produces antibodies reactive with the chlorinated dibenzo-p-dioxin, and recovering the produced antibodies from the selected hybridoma.

The present invention also provides an in vivo method of producing monoclonal antibodies reactive with a chlorinated dibenzo-p-dioxin by intraperitoneally placing in a histocompatible or immunosuppressed host a hybridoma that produces such antibodies and recovering the produced antibodies from the ascities fluid of the host.

The present invention also provides a method of producing a continuous cell line that produces monoclonal antibodies to a chlorinated dibenzo-p-dioxin by producing an immunogenic conjugate of the chlorinated dibenzo-p-dioxin and a macromolecule carrier, immunizing an animal with the conjugate, obtaining antibody-producing cells from the animal, fusing the antibody-producing cells with tumor cells to produce hybridomas, selecting from among the hybridomas a hybridoma that produces antibodies reactive with the chlorinated dibenzo-p-dioxin, and clonally expanding the selected hybridoma into a cell line.

The invention also provides a method for detecting the presence or concentration of 2,3,7,8-TCDD in a sample by adding monoclonal antibodies reactive with 2,3,7,8-TCDD to the sample and determining the presence or concentration of the 2,3,7,8-TCDD by an immunoassay, wherein the monoclonal antibodies are used as a reagent in the immunoassay.

The invention also provides a composition for determining the presence or concentration of 2,3,7,8-TCDD in a sample, which composition comprises an effective amount of a monoclonal antibody reactive with 2,3,7,8-TCDD in an acceptable carrier.

The invention also provides an immunological procedure for the isolation or removal of 2,3,7,8-TCDD in a sample, from a mixture on the basis of a selective immunological reaction with an antibody specific for the 2,3,7,8-TCDD, in which a monoclonal antibody reactive with 2,3,7,8-TCDD is used as the antibody.

The invention also provides a composition for isolating or removing 2,3,7,8-TCDD from a mixture containing it, which comprises an effective amount of a monoclonal antibody reactive with 2,3,7,8-TCDD immobilized on an acceptable matrix or in admixture with an acceptable carrier.

In a particularly preferred embodiment, the monoclonal antibodies of the present invention have the characteristics of the monoclonal antibodies produced by the hybridoma cell lines ATCC HB 9183 and ATCC HB 9184 or mutants or variants thereof. ATCC HB 9183 and ATCC HB 9184 are biologically pure cultures available from the permanent collection of the American Type Culture Collection (ATCC), 12301 Parklawn Drive, Rockville, Maryland, U.S.A. 20852 and were deposited on August 21, 1986. The immunoglobulins (antibodies) produced by these hybridomas are of the IgG1 isotype and subclass, as demonstrated by double gel immunodiffusion (Ouchterlony) analysis employing immunopurified anti-immunoglobulin class- and subclass-specific reagents. The monoclonality of the ATCC HB 9183 and ATCC HB 9184 antibodies was ensured by re-cloning the hybridomas which produced them.

The present invention also includes hybridomas and continuous cell lines that produce monoclonal antibodies to chlorinated dibenzo-p-dioxins. Preferably, those hybridomas and cell lines produce monoclonal antibodies to 2,3,7,8-TCDD, the chemical structure of which is depicted in Figure 1a. Most preferably, the continuous cell lines of the present invention have the characteristics of the hybridoma cell lines having ATCC Accession Numbers HB 9183 and HB 9184 or mutants or variants of those cell lines. The invention also encompasses individual cells of that cell line or any continuous cell line that produces the monoclonal antibodies of the present invention.

Persons skilled in the art can use known techniques to produce mutants or variants of ATCC HB 9183 or ATCC HB 9184. Such mutants or variants are encompassed within the present invention as long as they produce monoclonal antibodies reactive with 2,3,7,8-TCDD. In addition, persons skilled in the art, using techniques known in the art and the teachings disclosed herein, will be able to produce monoclonal antibodies reactive with 2,3,7,8-TCDD and hybridomas and cell lines that produce such monoclonal antibodies, which will have different characteristics from ATCC HB 9183 or ATCC HB 9184 or the antibodies produced by such cell lines. Nevertheless, such monoclonal antibodies and the hybridomas or cell lines producing them are within the scope of the present invention. The monoclonal antibodies produced by ATCC HB 9183 or ATCC HB 9184 will prevent other monoclonal antibodies that would otherwise react with 2,3,7,8-TCDD, which have been produced by other hybridomas, from so reacting with 2,3,7,8-TCDD. Such other monoclonal antibodies are encompassed within the scope of the present invention.

The hybridomas and continuous cell lines of the present invention may be produced by:

(a) producing an immunogenic conjugate of a macromolecule carrier and the particular isomer of chlorinated dibenzo-p-dioxin to which a monoclonal antibody is sought;

(b) immunizing an animal with the conjugate;

(c) obtaining antibody-producing cells from the animal;

(d) fusing the antibody-producing cells with tumor cells to produce hybridomas;

(e) selecting from among the hybridomas a hybridoma that produces antibodies reactive with the isomer of chlorinated dibenzo-p-dioxin.

The selected hybridoma or hybridomas may then be cloned to produce a cell line. The monoclonal antibodies of the present invention may be recovered from the selected hybridoma or hybridomas before or after they are expanded by cloning into a continuous cell line. As previously mentioned, in a preferred embodiment of the invention, the chlorinated dibenzo-p-dioxin is 2,3,7,8-TCDD. Thus, the preferred monoclonal antibodies are monoclonal antibodies to 2,3,7,8-TCDD.

In an alternative embodiment, the hybridomas of the present invention may be produced by the fusion of a tumor cell with any cell that produces antibodies to a chlorinated dibenzo-p-dioxin. The hybridomas produced by this embodiment can be used to produce the monoclonal antibodies of the invention by culturing them in a suitable medium and recovering the antibodies from the medium.

Substances with molecular weights of approximately 1000 or less, such as 2,3,7,8-TCDD (molecular weight 455), do not ordinarily induce the production of antibodies; i.e., they are non-immunogenic. However, such substances can be chemically attached to larger immunogenic carrier molecules, such as a carbohydrate or a protein, in order to induce the production of antibodies against the smaller substance. Such antibodies often bind only to the substances conjugated to the carrier, but with appropriate conjugation and selection methods, monoclonal antibodies that bind to the free or unbound substance can be prepared and identified.

The general techniques for producing an immunogenic conjugate of a chlorinated dibenzo-p-dioxin and a macromolecule carrier are known in the art. For example, see U.S. Patent Specification No. 4,456,691 (Stark) issued June 26, 1984, and Albro et al., Toxicol. Appl. Pharmacol. 50, 137-146 (1979).

The macromolecule may be attached to the chlorinated dibenzo-p-dioxin at any of the substituted or unsubstituted carbon positions of the molecule. Once the immunogenic conjugate has been produced, it is used to immunize an animal host by techniques known in the art. Such techniques usually involve inoculation, but they may involve other modes of administration. A sufficient amount of the conjugate is administered to create an immunogenic response in the animal host.

Any host that produces antibodies to the conjugate may be used. Conventionally used animals include rabbits and rodents, such as rats or mice. Mice are preferred for the present invention.

Once the animal has been immunized and sufficient time has passed for it to begin producing antibodies to the conjugate, antibody-producing cells are recovered. Although any antibody-producing cells may be used, B lymphocytes obtained from the animal's spleen are preferred.

The antibody-producing cells are fused with tumor cells to produce hybridomas. As used herein, the term "tumor cell" includes any cell that is capable of fusing with an antibody-producing cell to create a hybrid "immortal" cell; i.e., one which is capable of continuous growth in vitro. Preferred tumor cells are antibody-producing cells which have been transformed and which have lost their ability to produce immunoglobulin. Such cells include myeloma cells and mouse plasmacytoma cells. Particularly preferred are mouse plasmacytoma cells that are deficient in the enzyme hypoxanthine-guanine phosphoribosyl transerase (HGPRT), which allows the selection of hybridomas from unfused antibody producing cells or plasmacytoma cells when grown on a medium containing hypoxanthine, aminopterin, and thymidine.

Various tumor cells useful for fusing with antibody-producing cells are known and readily available to those skilled in the art. One such type of cell is the mouse plasmacytoma cell line P3-X63-Ag8.653, described by Kearney, J. Immunology, 123, 1548 (1979) which is incorporated herein by reference. This cell line is available from American Type Culture Collection, Rockville, Maryland, where it is designated ATCC CRL-1580.

It should be noted that the antibody-producing cell and the tumor cell can be from different animal species. For example, see Nowinski et al., Science, 210:537 (1980).

As mentioned previously, once the cells have been fused, it is necessary to separate the hybridomas from the unfused cells. The antibody-producing cells will normally die after a few days in culture, but the tumor cells are "immortal". However, by using tumor cells that are deficient in HGPRT and growing the fused cells on a medium containing hypoxanthine, aminopterin, and thymidine, the hybridomas will be naturally selected for, since the tumor cells are unable to survive on such a medium. However, other known selection techniques may also be used.

After the hybridomas are selected, they are evaluated to determine which are producing antibodies to the chlorinated dibenzo-p-dioxin. Various immunoassays known to those skilled in the art may be used to evaluate the culture supernatants of the hybridomas. Care must be taken to identify hybridomas that are producing monoclonal antibodies only to the chlorinated dioxin rather than to the dioxin-macromolecure carrier. That is, the monoclonal antibodies which are desired, useful, and provided by this invention are those that react with free, i.e., unconjugated or unbound, chlorinated dioxins.

The preferred selection technique is a competitive inhibition enzyme immunoassay that evaluates the ability of a free chlorinated dioxin, such as 2,3,7,8-TCDD, to inhibit the binding of monoclonal antibodies to the dioxin/protein carrier. The technique is a modification of the one disclosed in Hunter et al., FEBS Lett. 149:147-151 (1982). The modification comprises the preparation of a screening conjugate with the dioxin molecule conjugated to the macromolecular carrier at a position different from that used to prepare the immunizing conjugate. By employing such a heterologous screening conjugate, it was reasoned that any monoclonal antibodies raised against the immunizing conjugate would bind less strongly to the heterologous screening conjugate, thereby affording the free chlorinated dioxin a competitive advantage. In the Examples below, GIgG 2-adipamidoy1,3,7,8-trichlorodibenzo-p-dioxin was used to immunize mice and BSA-1-adipamidoyl,3,7,8-trichlorodibenzo-p-dioxin was used for screening (see Figures 1b and 1c respectively).

Once the monoclonal antibody-producing hybridomas have been selected, the antibodies can be recovered from such hybridomas by known techniques. Generally, it is useful to clone one or more of the monoclonal antibody-producing hybridomas to expand it into a continuous cell line that can be used to produce the monoclonal antibodies in quantity.

The previously mentioned methods of producing the monoclonal antibodies of the present invention are in vitro methods. The present invention also comprises an in vivo process for producing monoclonal antibodies to chlorinated dibenzo-p-dioxins. Such antibodies are produced by placing a hybridoma that produces the antibodies intraperitoneally in a histocompatible or immunosuppressed host. This causes the host to produce ascites tumors which, in turn, produce a fluid that contains the monoclonal antibodies produced by the hybridoma. After a sufficient time has passed for the antibodies to have been produced in sufficient quantities, they may be recovered by known techniques. This method is particularly suitable for producing the monoclonal antibodies in commercially useful quantities.

Just as a variety of different systems and methods might be employed to produce monoclonal antibodies reactive with chlorinated dibenzo-p-dioxins, so do a variety of monoclonal antibodies result from these measures that are distinct from the antibody illustrated in the Examples below. However, such monoclonal antibodies, whose production is enabled by the teachings herein, are still clearly within the scope of this invention. The salient feature of such antibodies, for the purposes of this invention, besides their monoclonality, is their reactivity in any way with chlorinated dibenzo-p-dioxins, regardless of the species of origin, isotype, molecular specificity, affinity, method of production, or particular type of hybridoma employed in its production.

The monoclonal antibodies of the present invention may be used to identify chlorinated dibenzo-p-dioxins, particularly 2,3,7,8-TCDD, in materials and to determine the concentration of the chemical in those materials. Such materials include, for example, soil, water, and body fluids. When used as a reagent in various immunoassays for determining the presence or concentration of 2,3,7,8-TCDD, the monoclonal antibodies of the present invention provide an improved assay. Detection is more convenient, rapid, sensitive, and specific. The immunoassays in which the monoclonal antibodies of the present invention may be used include, but are not limited to, radioimmunoassay, competition immunoprecipitation assay, enzyme-linked immunoabsorbent assay, and immunofluorescence assay.

A composition for determining the presence or concentration of 2,3,7,8-TCDD in material in accordance with the present invention contains a concentration of the antibody effective to detect the presence of the chemical or to quantify its amount. The antibody can be mixed with or attached to any suitable carrier, such as a latex particle or plastic microtiter plate. It may also be conjugated with an enzyme or dye or radio-labelled, depending upon what immunological method is employed. Consequently, any assay system which employs monoclonal antibodies reactive with chlorinated dibenzo-p-dioxins, including 2,3,7,8-TCDD, is embraced by this invention.

The monoclonal antibodies of this invention are also useful for the isolation, purification, neutralization, and/or removal of chlorinated dioxins from complex mixtures or solutions on the basis of a selective immunological reaction. The use of a monoclonal antibody reactive with a chlorinated dioxin represents an improvement over the known methods. The novel attributes of these monoclonal antibodies which make them particularly useful for these applications are their great specificity, compared with polyclonal antibodies, and their availability in virtually limitless quantities, which permits their use on a large, industrial or commercial scale.

For example, monoclonal antibodies to 2,3,7,8-TCDD are useful to separate and purify 2,3,7,8-TCDD from a mixture of other chlorinated dioxins or similar organic compounds. The mixture is brought into contact with immobilized monoclonal antibodies to 2,3,7,8-TCDD, which separates the 2,3,7,8-TCDD from the mixture by forming immobilized complexes of the 2,3,7,8-TCDD bound to the antibody. After the mixture is removed, the 2,3,7,8-TCDD is separated from the antibodies and recovered in purified form by known techniques.

A composition in accordance with the invention useful for purifying or removing a chlorinated dioxin from complex mixtures contains an effective amount of the monoclonal antibody of this invention, immobilized on an acceptable matrix or in admixture with an acceptable carrier, to permit reaction and binding with the chlorinated dioxin.

The monoclonal antibodies of this invention are also useful reagents for research related to the structure and function of chlorinated dioxins, particularly 2,3,7,8-TCDD. Their exquisite specificity allows them to be used for immunochemical and structure-activity analyses of these chemicals, and makes them more useful in these applications than less specific, conventional, polyclonal antibodies.

A composition in accordance with the present invention useful as an investigational reagent contains an amount of antibody effective in providing information upon mixture with a chlorinated dioxin and subsequent analysis. Determination of the amount of antibody necessary to accomplish a particular research goal depends upon the specific types of investigation involved, and it is readily within the skill of one carrying out such research.

The preparation of immunizing and screening conjugates was carried out as follows:

Production of an Immunogenic Protein/Dioxin Conjugate

The method of preparing an immunogenic protein/dioxin conjugate was modified from that described by Albro et al., Toxical. Appl. Pharmacol., 50, 137-146 (1979).

Production of 2-amino-3,7,8-trichlorodibenzo-p-dioxin

2-Amino-3,7,8-trichloro-dibenzo-p-dioxin (Compound I) was produced by the reduction of 2-nitro-3,7,8-trichloro dibenzo-p-dioxin (Compound II). An ethanolic solution of Compound II (6 mg/ml) was added to a refluxing mixture of 5 ml hydrazine hydrate in 10 ml of 95% ethanol containing 80 mg of palladium on charcoal. The material was refluxed for 2 hours, concentrated, and tested by thin layer chromatography, using silica gel with a fluorescent indicator. Both the starting nitro compound (II) and the reduced material (I) gave blue spots when visualized by spraying with a freshly prepared solution made by mixing 0.1 M ferric chloride and 0.1 M potassium ferricyanide in equal parts by volume.

The product was purified by ether-water (pH 11) partitioning three times. The ether extract was treated to remove water and concentrated to dryness. Final purification was by preparative thin layer chromatography using chloroform-methanol (3:1, volume:volume) as a solvent and elution from gel scrapings with ether.

Reaction of 2-amino-3,7,8-trichlorodibenzo-p-dioxin With Methyladipoyl Chloride

Methyladipoyl chloride was prepared according to Durham et al., Organic Synthesis, vol. 4, p. 556 (1963), incorporated herein by reference, and vacuum distilled. An ether solution of Compound I was added to a reaction flask and taken to dryness under a stream of nitrogen. 5 ml of dry pyridine was added, and the flask was brought to ice temperature with stirring. 0.5 ml of methyladipoyl chloride was added, the flask was equipped with a drying tube and the reaction was allowed to proceed for 1 hour. It was then placed in the refrigerator overnight.

20 ml of diethyl ether was added to the reaction mixture, and it was filtered. The filtrate was extracted with 1 N hydrochloric acid, 0.4 M $Na_2CO_3$, and saturated NaCl and dried. Thin layer chromatography of the ether solution on silica gel with benzene as a solvent showed no residual Compound I. The product, 2-methyladipamidoyl-3,7,8-trichlorodibenzo-p-dioxin (Compound III) had a higher $R_f$ than Compound I and gave no reaction with ferric chloride/potassium ferricyanide spray.

Hydrolysis of the Methyl Ester from 2-methyladipamidoyl-3,7-8-trichlorodibenzo-p-dioxin

The ether solution of Compound III was added to a flask equipped with a reflux condensor and taken to dryness under nitrogen. It was taken up in 10 ml 95% ethanol and brought to reflux. 4.7 ml of 0.3 N sodium hydroxide was added. The mixture was allowed to reflux for 90 minutes, after which it was acidified and extracted with ether. The ether extract was washed with water and dried.

Mixed Anhydride Formation

The product of the hydrolysis, 2-adipamidoyl-3,7,8-trichlorodibenzo-p-dioxin, was added to a reaction flask, taken to dryness, and dissolved in 2.4 ml peroxide free dioxane. 15 microliters of tri-n-butyl amine were added. The solution was stirred for 5 minutes at room temperature and 10 ml of fresh isobutylchloroformate were added. Stirring was continued for 20-30 more minutes.

Conjugation to Goat Immunoglobulin G

50 mg of goat immunoglobulin G was dissolved in 5.45 ml of 0.037 N sodium hydroxide solution and cooled in an ice bath. 4.25 to 5 ml of dry, peroxide-free dioxane was added to this solution and the total mixed anhydride solution was added dropwise. Stirring was continued for 1 hour. An additional 0.1 ml of sodium hydroxide was added to counteract the reduction in pH caused the progress of the reaction. Stirring was continued for an additional 3 hours. The reaction was confirmed by the shift in pH from 9-10 to 7-8 with time.

The structure of the immunogen is shown in Figure 1b. It can be seen that the attachment to the protein is through the 2-position on the TCDD molecule. It is possible, using similar chemistry, to attach the TCDD molecule to the protein through the 1-position, or indeed, to any position on the TCDD molecule.

To calculate the number of moles of 2,3,7,8-TCDD bound per mole of protein (epitope density) a difference spectrum was run. Equimolar volumes of GIgG 2-adipamidoyl,3,7,8-trichlorodibenzo-p-dioxin and normal GIgG (in 0.1 N NaOH) were placed in the sample and reference cuvettes of a UV spectrophotometer. A difference curve was run at 302 nm, the difference in optical density attributable to the 2-adipamidoyl,3,7,8-trichlorodibenzo-p-dioxin. The extinction coefficient (ε) for 2-adipamidoyl,3,7,8-trichlorodibenzo-p-dioxin at 302 nm is $3.83 \times 10^3$ L $cm^{-1}$ $mol^{-1}$ using the formula

$$\text{molar concentration} = \frac{\text{O.D. } 302 \text{ nm}}{\xi \;\; 302 \text{ nm}} \text{ (1 cm cuvettes)}$$

Preparation of Protein/Dioxin Conjugate Used for Screening

The screening conjugate was prepared using the same procedure as described for producing the immunogenic conjugate, except that the starting material was 1-nitro3,7,8-trichlorodibenzo-p-dioxin and the protein to which it was conjugated was bovine serum albumin (BSA). The structure of the screening conjugate can be seen in Figure Ic.

The invention will now be illustrated with reference to the following Examples which describe the preparation of hybridomas that produce monoclonal antibodies reactive with 2,3,7,8-TCDD, how clones of these cells were reproduced on a large scale, and how monoclonal antibodies were obtained from these clones. Specifically, the examples describe how spleen cells (B lymphocytes) obtained from mice previously immunized with GIgG-2-adipamidoyl,3,7,8-trichlorodibenzo-p-dioxin were fused with mouse plasmacytoma cells, resulting in hybridomas, and how cells from these hybridomas, which secreted monoclonal antibodies to 2,3,7,8-TCDD, were cloned. They also describe how one distinct hybridoma clone was reproduced on a large scale employing both in vitro and in vivo techniques, and how monoclonal antibodies reactive with 2,3,7,8-TCDD were obtained from these hybridomas in large, usable quantities.

Example 1

Production of Hybridomas

Six week old female BALB/c mice were injected subcutaneously at four weekly intervals with 50 micrograms of purified GIgG-2-adipamidoyl,3,7,8-trichlorodibenzo-p-dioxin suspended in 0.15 M NaCl. The donor mice were killed by cervical dislocation three days following the last immunization; the spleen was removed aseptically and placed in a 35 mm plastic Petri dish with 5 ml of cold Dulbecco's Minimal Essential Medium (DMEM). The spleen was dissociated into a single cell suspension, the cells were washed twice with cold DMEM, and resuspended in the same medium.

An immunoglobulin non-secreting mouse plasmacy toma cell line (P3-X63-Ag8.653) deficient in the enzyme hypoxanthine-guanine phosphoribosyl transferase (HGPRT-, EC 2.4.2.8), as disclosed by Kearney, Journal of Immunology, 123:1548, 1979, which is incorporated herein by reference, was used as the fusion partner. This cell line is available from the American Type Culture Collection, Rockville, Maryland, where it is designated ATCC CRL-1580. The plasmacytoma cell line was maintained in DMEM containing 10% fetal bovine serum and further supplemented with 2 mM L-glutamine, 1% sodium pyruvate, 1% non-essential amino acids, 100 IU/ml penicillin, and 100 micrograms per milliliter streptomycin. For three days prior to fusion, 0.1 mM 8-azaguanine was added to the plasmacytoma cells in order to kill any HGPRT+ revertants. On the day of fusion, the plasmacytoma cells were harvested from 75 $cm^2$ culture flasks, washed once, and resuspended in serum-free DMEM. The plasmacytoma and previously harvested spleen cells were counted and their viability assessed by Trypan blue dye exclusion.

The fusion technique used was modified from that of Gefter et al., Somatic Cell Genetics, 3:231, 1977, which is hereby incorporated by reference. Described below is the fusion experiment which yielded ATCC HB 9183; other fusion experiments performed in an identical manner produced other hybridoma clones, including ATCC HB 9184.

To a sterile 50 ml conical plastic tube was added $1.0 \times 10^8$ spleen cells and $1.0 \times 10^7$ plasmacytoma cells. The plasmacytoma-spleen cell suspension was centrifuged at 250 × g for 10 minutes at room temperature and the medium then decanted to near dryness. The cell pellet was loosened gently by flicking and 1 ml of 50% polyethylene glycol (MW 1400) in DMEM, without serum, was added in drops over 45 seconds. The tube was gently agitated during this process. One minute later, another 2 ml of DMEM was added over 2 minutes. An additional 20 ml of DMEM was added over the next 2 minutes, and the cells pelleted by centrifugation at 250 × g for 10 minutes at room temperature.

The fluid was decanted and 40 ml of enriched selection medium were added. This medium was composed of DMEM containing 10% fetal bovine serum and supplemented with 2mM L-glutamine, l% sodium pyruvate, l% non-essential amino acids, 100 IU/ml penicillin, 100 micrograms per milliliter streptomycin, 1 mM oxaloacetic acid, 10% NCTC 109, and 0.2 micrograms per milliliter bovine pancreatic insulin. The medium also contained $1.0 \times 10^{-4}$ M hypoxanthine, $4.0 \times 10^{-7}$ M aminopterin, and $1.6 \times 10^{-5}$ M thymidine (HAT). Aminopterin is toxic for cells that lack the enzyme HGPRT and therefore kills all unfused plasmacytoma cells. Fused cells (hybridomas) survive in HAT because they obtain HGPRT from the B lymphocyte (spleen cell) fusion partner.

Example 2

Selection of Hybridomas Producing Monoclonal Antibodies to 2,3,7,8-TCDD

Aliquots of 0.2 ml containing $5.0 \times 10^5$ cells were transferred from the mixture produced in Example 1 into individual wells of several sterile flat-bottomed microtiter plates. The plates were incubated at 37°C in a humidified atmosphere consisting of 6% $CO_2$ and 94% air. Fresh selection medium was added on alternate days for the next 11 days.

On day 11, the microculture supernatants were tested for antibodies reactive with 2,3,7,8-TCDD by the following enzyme immunoassay (EIA). Purified BSA-1-adipamidoyl,3,7,8-trichlorodibenzo-p-dioxin conjugate (see Fig. 1c) was dissolved at a concentration of 5 micrograms per milliliter in coating buffer (phosphate buffered saline, PBS, pH 7.4) and dispensed in 50 microliter aliquots into 96-well polyvinyl chloride microtiter plates (Dynatech Laboratories, Inc., Chantilly, VA). After overnight incubation at 4°C, the conjugate solution was removed and the wells washed five times with PBS-Tween (0.5 ml/l Tween-20). 50 microliter test samples were added to the wells, the plates incubated at 4°C for 30 minutes, and then washed five times with PBS-Tween. The final two steps, separated by PBS-Tween washes, were as follows: addition of 50 microliters of a 1:500 dilution of goat anti-mouse IgG/IgM conjugated with the enzyme alkaline phosphatase (Sigma Chemical Co., St. Louis, MO) followed by incubation at 4°C for 30 minutes; then the addition of 50 microliters of p-nitrophenyl phosphate (Sigma-104) substrate, 1 mg/ml in 10% diethanolamine, pH 9.8, and incubation at 25°C for 30 minutes. Absorbance was read at 405 nm in a Bio-Tek micro-EIA spectrophotometer (Bio-Tek Instruments, Inc., Winooski, VT).

Furthermore, to verify that the antibodies were truly reactive with 2,3,7,8-TCDD, and not only with the BSA-2-adipamidoyl,3,7,8-trichlorodibenzo-p-dioxin conjugate, the culture supernatants were preincubated with $1.0 \times 10^{-4}$ M 2,3,7,8-TCDD. This so-called competitive inhibition enzyme immunoassay (CIEIA) results in the inhibition of antibody binding to the solid phase-bound conjugate by fluid phase 2,3,7,8-TCDD. Whereas 58 of 384 cultures were positioned by EIA (15%), only 6 were positioned by CIEIA (1.5%, showing at least 50% inhibition with $1.0 \times 10^{-4}$ M 2,3,7,8-TCDD). Positive cultures were expanded in number and transferred to 35 $cm^2$ flasks. The medium from these cultures was retested, and those maintaining antibody secretion were cryopreserved and stored at -179°C in liquid nitrogen vapor phase.

Example 3

Cloning of Hybridoma Culture that Produces Monoclonal Antibodies to 2,3,7,8-TCDD

One of the positive hybridoma cultures described in Example 2 was selected for cloning by limiting dilution. 100 microliter aliquots containing 0-1 cells were transferred to several hundred individual wells in sterile flat-bottomed microtiter plates that had been previously seeded with mouse thymocytes which serve as "feeder" cells for the hybridomas. After 14 days, the cloned cultures were tested again by EIA and CIEIA for antibodies reactive with 2,3,7,8-TCDD, and one positive clone was selected for further study. This cloned hybridoma, which has been deposited in the American Type Culture Collection under accession number ATCC HB 9183, was expanded in numbers, cryopreserved, and stored in the same manner as the parental cells lines from which it was derived.

Demonstration of Reactivity of Monoclonal Antibodies with 2,3,7,8-TCDD

ATCC HB 9183 was tested for antibodies reactive with 2,3,7,8-TCDD by EIA and CIEA as described in Example 2. In the EIA, the microtiter plate was coated with 5 micrograms per milliliter of BSA-1-adipamidoyl,3,7,8-trichlorodibenzo-p-dioxin conjugate. A titration curve for the ATCC HB 9183 antibodies from culture supernatants is shown in Fig. 2.

To verify that the antibodies interacted with free TCDD, a CIEIA was performed. This assay was similar to the straight EIA described in Example 2 except that the antibodies were preincubated for 1 hour with various concentrations of 2,3,7,8-TCDD. Interaction of the antibodies with 2,3,7,8-TCDD in solution inhibits their binding to conjugate on the solid phase, thereby decreasing the ultimate color reaction. Various concentrations of TCDD were added to glass tubes in benzene, then blown dry under nitrogen. A 1:5 dilution of ATCC HB 9183 from culture supernatant was added in PBS, incubated 1 hour at room temperature, the mixture added to a coated microtiter plate. The linear curve indicated in Figure 3 demonstrates that the ATCC HB 9183 antibody binds directly to free TCDD, and the curve also indicates that utility of the antibody for quantifying levels of TCDD in unknown samples.

Example 4

In Vitro Production of Monoclonal Antibodies to 2,3,7,8-TCDD

Stationary cultures of ATCC HB 9183 were grown in 75 $cm^2$ culture flasks containing DMEM supplemented as described in Example 1. After 5-7 days of incubation at 37°C under 6% $CO_2$, 1-2 liters of culture fluid containing approximately 10 micrograms per milliliter of anti-2,3,7,8-TCDD antibody were obtained.

Example 5

In Vivo Production of Monoclonal Antibodies to 2,3,7,8-TCDD

An in vivo method for obtaining large amounts of monoclonal antibodies involved the adaptation of ATCC HB 9183 to grow as an "ascites" tumor. Female BALB/c mice were "primed" by intraperitoneal injection of 0.5 ml of pristane (2,6,10,14-tetra-methylpentadecane). Pristane is a sterile irritant which elicits a serous secretion ("ascites") in the peritoneal cavity of mice which acts as a growth medium. Approximately 7-10 days following the pristane injection, aliquots containing $1.0 \times 10^6$ actively growing hybridoma cells harvested from in vitro cultures as described in Example 4 were inoculated into the peritoneal cavities of primed mice. Hybridoma cells were serially passaged at 1-2 weekly intervals to freshly primed mice. After 3-4 such passages, the ATCC HB 9183 hybridomas became well-adapted ascites tumors, growing rapidly in the fluid microenvironment of the mouse peritoneal cavity and secreting large quantities (e.g., 2-10 mg/ml) of monoclonal antibodies reactive with 2,3,7,8-TCDD. Routinely, 5-10 ml of ascites fluid was removed from each mouse by aspiration. The antibody-secreting tumor cells were separated from the antibody-containing fluid phase and reinjected into other primed mice, and the process was repeated.

**Claims**

1. A method of producing monoclonal antibodies reactive with a chlorinated dibenzo-p-dioxin characterized by producing an immunogenic conjugate of said chlorinated dibenzo-p-dioxin and a macromolecule carrier; immunizing an animal with said conjugate; obtaining antibody-producing cells from said animal; fusing said cells with tumor cells to produce hybridomas; selecting from among said hybridomas at least one hybridoma that produces antibodies reactive with said chlorinated dibenzo-p-dioxin; and recovering said produced antibodies from said selected hybridoma.

2. A method according to claim 1 characterized in that the chlorinated dibenzo-p-dioxin is 2,3,7,8-tetrachlorodibenzo-p-dioxin.

3. A method according to claim 1 or 2, characterized in that the immunized animal is a rodent.

4. A method according to claim 1, 2 or 3, characterized in that the tumor cells are mouse plasmacytoma cells.

5. A method according to claim 1, 2, 3 or 4, characterized in that the hybridomas have the characteristics of ATCC HB 9183 or ATCC HB 9184 or mutants or variants thereof.

6. A method of producing monoclonal antibodies reactive with 2,3,7,8-tetrachlorodibenzo-p-dioxin (2,3,7,8-TCDD) characterized by producing an immunogenic conjugate of said 2,3,7,8-TCDD and a protein; immunizing a mouse with said conjugate; recovering antibody-producing cells from the spleen of said mouse; fusing said antibody-producing cells with mouse plasmacytoma cells deficient in the enzyme hypoxanthine-guanine phosphoribosyl transferase to form hybridomas; selecting at least one of said hybridomas by growth in a medium comprising hypoxanthine, aminopterin, and thymidine; identifying at least one of said hybridomas that produces an antibody to said 2,3,7,8-TCDD in free or unconjugated form; culturing said identified hybridoma to produce said antibody in a recoverable quantity; and recovering the antibodies produced by said cultured hybridoma.

7. A method of producing monoclonal antibodies to 2,3,7,8-tetrachlorodibenzo-p-dioxin characterized by culturing a hybridoma having the characteristics of ATCC HB 9183 or ATCC HB 9184 or mutants or variants thereof in a culture medium and recovering said antibodies from said medium.

8. An in vivo method of producing monoclonal antibodies reactive with a chlorinated dibenzo-p-dioxin characterized by intraperitoneally placing in a histocompatible or immunosuppressed host a hybridoma that produces said antibodies; and recovering the produced antibodies from the ascites fluid of said host.

9. A method according to claim 8, characterized in that the chlorinated dibenzo-p-dioxin is 2,3,7,8-tetrachlorodibenzo-p-dioxin.

10. A method according to claim 8 or 9 characterized in that the host is a rodent.

11. A method according to claim 8, 9 or 10, characterized in that the hybridoma has the characteristics of ATCC HB 9183 or ATCC HB 9184 or mutants or variants thereof.

12. A method of producing a continuous cell line that produces monoclonal antibodies to a chlorinated dibenzo-p-dioxin characterized by producing an immunogenic conjugate of said chlorinated dibenzo-p-dioxin and a macromolecule carrier; immunizing an animal with said conjugate; obtaining antibody-producing cells from said animal; fusing said antibody-producing cells with tumor cells to produce hybridomas; selecting from among said hybridomas a hybridoma that produces antibodies reactive with said chlorinated dibenzo-p-dioxin; and clonally expanding said selected hybridoma into a cell line.

13. A method according to claim 12, characterized in that the chlorinated dibenzo-p-dioxin is 2,3,7,8-tetrachloro-dibenzo-p-dioxin.

14. A hybridoma which produces a monoclonal antibody characterized in that said antibody is reactive with a chlorinated dibenzo-p-dioxin.

15. A hybridoma according to claim 14, characterized in that the chlorinated dibenzo-p-dioxin is 2,3,7,8-tetrachlorodibenzo-p-dioxin.

16. A hybridoma according to claim 14 or 15, characterized in that said hybridoma has the characteristics of ATCC HB 9183 or ATCC HB 9184 or mutants or variants thereof.

17. A monoclonal antibody characterized in that said antibody is reactive with a chlorinated dibenzo-p-dioxin.

18. An antibody according to claim 17, characterized in that the chlorinated dibenzo-p-dioxin is 2,3,7,8-tetrachlorodibenzo-p-dioxin.

19. A monoclonal antibody characterized in that said antibody is recovered from the cell line produced by the method of claim 12 or 13.

20. An antibody according to claim 17 or 18, characterized in that said antibody is recovered from a hybridoma.

21. An antibody according to claim 20, characterized in that the hybridoma has the characteristics of ATCC HB 9183 or ATCC HB 9184 or mutants or variants thereof.

22. A method for determining the presence or concentration of 2,3,7,8-tetrachlorodibenzo-p-dioxin (2,3,7,8-TCDD) in a sample by employing an immunoassay, characterized by using a monoclonal antibody reactive with said 2,3,7,8-TCDD as a reagent in said immunoassay.

23. A method for detecting the presence or concentration of 2,3,7,8-TCDD in a sample characterized by adding monoclonal antibodies reactive with said 2,3,7,8-TCDD to said sample; and determining the presence or concentration of said 2,3,7,8-TCDD by an immunoassay in which said monoclonal antibodies are used as a reagent.

24. An immunological procedure for the isolation or removal of 2,3,7,8-TCDD from a mixture on the basis of a selective immunological reaction with an antibody specific for said 2,3,7,8-TCDD, characterized by using a monoclonal antibody.

25. A method for the purification of 2,3,7,8-TCDD characterized by contacting material containing 2,3,7,8-TCDD with immobilized monoclonal antibodies reactive with said 2,3,7,8-TCDD to separate the latter from said material by forming immobilized complexes of said antibody and said 2,3,7,8-TCDD; and separating said 2,3,7,8-TCDD from said immobilized antibodies to recover said 2,3,7,8-TCDD in purified form.

26. A method for the biochemical, immunological, functional, or other investigational analysis of 2,3,7,8-TCDD, characterized by using a monoclonal antibody reactive with said 2,3,7,8-TCDD and in an amount effective to result in said analysis.

27. A composition for determining the presence or concentration of 2,3,7,8-TCDD in a sample characterized in that said composition comprises an effective amount of a monoclonal antibody reactive with said 2,3,7,8-TCDD and in an acceptable carrier.

28. A composition for isolating or removing 2,3,7,8-TCDD from a mixture containing 2,3,7,8-TCDD characterized in that said composition comprises an effective amount of the monoclonal antibody reactive with said 2,3,7,8-TCDD and immobilized on an acceptable matrix or in admixture with an acceptable carrier.

29. A composition for the purification of 2,3,7,8-TCDD characterized in that said composition comprises a monoclonal antibody reactive with said 2,3,7,8-TCDD and immobilized on an acceptable support or carrier.

30. A composition for the biochemical, immunological, functional, or other investigational analysis of 2,3,7,8-TCDD characterized in that said composition comprises an effective amount of a monoclonal antibody reactive with said 2,3,7,8-TCDD and in an acceptable carrier.

*Fig. 1a*

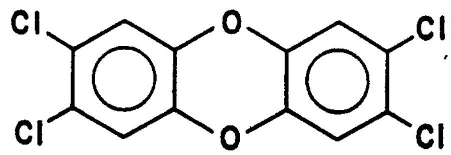

2,3,7,8-tetrachlorodibenzo-p-dioxin

*Fig. 1b*

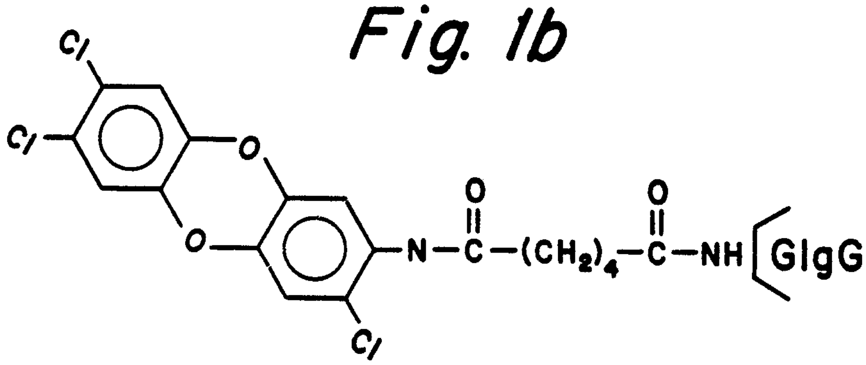

2-adipamidoyl,3,7,8-trichlorodibenzo-p-dioxin

*Fig. 1c*

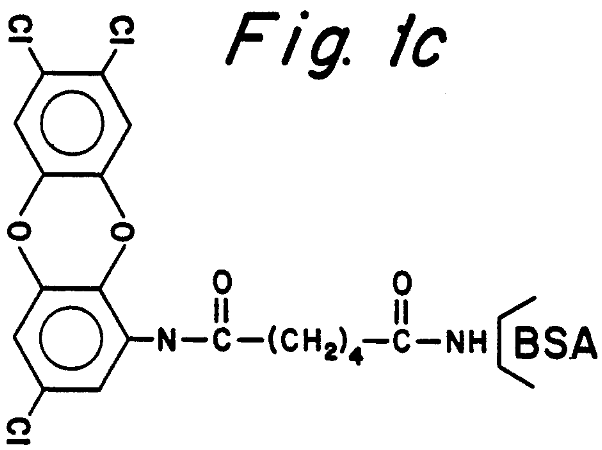

1-adipamidoyl,3,7,8-trichlorodibenzo-p-dioxin

0258006

Fig. 2

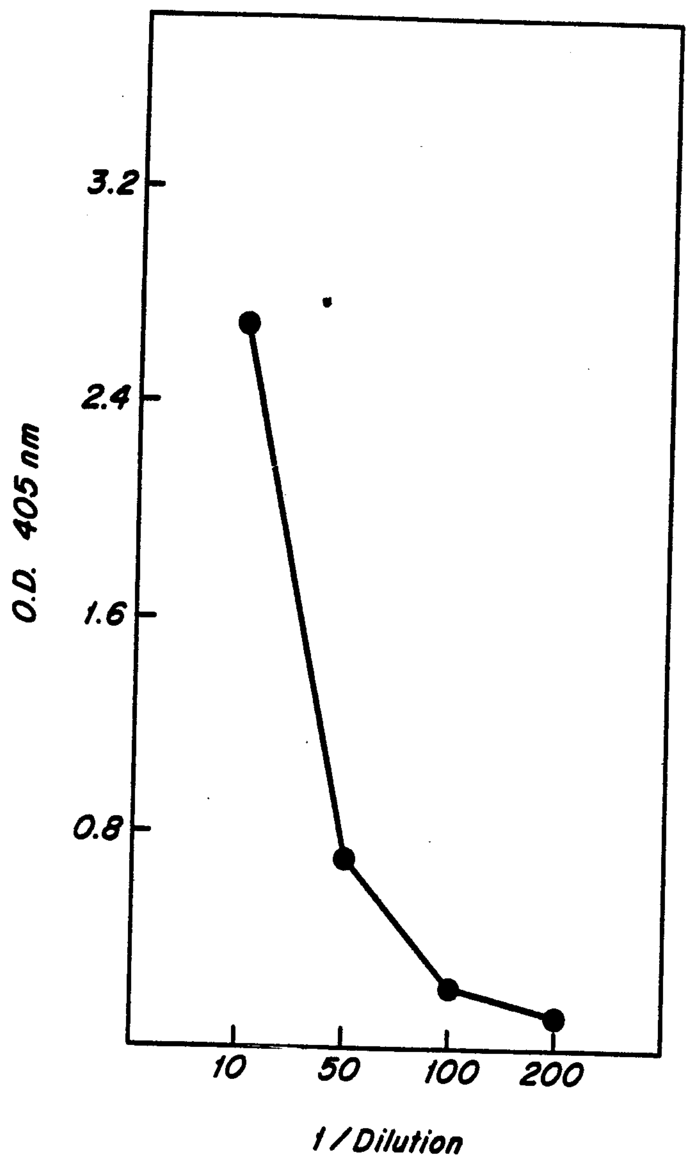

0258006

Fig. 3